# EUROPÄISCHE PATENTANMELDUNG

(11) **EP 0 985 447 A2**
(43) Veröffentlichungstag der Anmeldung: **15.03.2000**
(21) Anmeldenummer: 99116663.8
(22) Anmeldetag: 26.08.1999
(51) Int. Cl.: B01J 23/50, B01J 23/66, C07C 5/09

(54) **Katalysator und Verfahren zur Selektivhydrierung ungesättigter Verbindungen in Kohlenwasserstoffströmen**

(30) Priorität: 04.09.1998 DE 19840373
(71) Anmelder: BASF AKTIENGESELLSCHAFT, 67056 Ludwigshafen (DE)
(72) Erfinder: Frenzel, Andrea, Dr., 67117 Limburgerhof (DE); Freire Erdbrügger, Cristina, Dr., 67251 Freinsheim (DE); Schwab, Ekkehard, Dr., 67434 Neustadt (DE); Hesse, Michael, Dr., 67549 Worms (DE); Linden, Gerd, Dr., 69120 Heidelberg (DE); Wanjek, Herbert, Dr., 67133 Maxdorf (DE); Allmann, Hans-Martin, 74867 Neunkirchen (DE)

(57) **Zusammenfassung**

Ungesättigte Verbindungen in Kohlenwasserstoffströmen werden an einem Katalysator hydriert, der mindestens ein Metall der 10. Gruppe des Periodensystems der Elemente und mindestens ein Metall der 11. Gruppe des Periodensystems der Elemente auf einem Aluminiumoxidträger enthält, wobei das Metall oder die Metalle der 10. Gruppe im wesentlichen in einer oberflächennahen Randschicht des Katalysatorkorns konzentriert ist oder sind, das Metall oder die Metalle der 11. Gruppe im wesentlichen gleichmäßig über das Volumen des Katalysatorkorns verteilt vorliegt oder vorliegen, und das Gewichtsverhältnis des Metalls oder der Metalle der 11. Gruppe zum Metall oder zu den Metallen der 10. Gruppe höchstens 1,95 beträgt.

## Beschreibung

Die vorliegende Erfindung betrifft edelmetallhaltige Katalysatoren auf einem Aluminiumoxidträger und Verfahren zur Selektivhydrierung ungesättigter Verbindungen in diese enthaltenden Kohlenwasserstoffströmen unter Verwendung dieser Katalysatoren. Insbesondere betrifft die vorliegende Erfindung edelmetallhaltige Katalysatoren auf einem Aluminiumoxidträger und Verfahren zur Selektivhydrierung von Alkinen und/oder Alkadienen in diese enthaltenden C2- oder C3-Kohlenwasserstoffströmen.

In Raffinerien und petrochemischen Anlagen werden in großem Umfang Kohlenwasserstoffströme erzeugt, gelagert und verarbeitet. In diesen Kohlenwasserstoffströmen sind häufig ungesättigte Verbindungen vorhanden, deren Anwesenheit insbesondere bei Verarbeitung und/oder Lagerung bekanntermaßen zu Problemen führt, oder die nicht das gewünschte Wertprodukt darstellen, und die daher unerwünschte Komponenten der entsprechenden Kohlenwasserstoffströme sind. Allgemeine Übersichten über derartige Probleme bei Steamcrackern und übliche Lösungen gaben beispielsweise H.-M. Allmann, Ch. Herion und P. Polanek in ihrem Vortrag "Selective Hydrogenations and Purifications in the Steamcracker Downstream Treatment" auf der DGMK-Konferenz "Selective Hydrogenation and Dehydrogenation" am 11. und 12. November 1993 in Kassel, Deutschland, dessen Manuskript auch im Tagungsbericht 9305 der DGMK Deutsche Wissenschaftliche Gesellschaft für Erdöl, Erdgas und Kohle e. V., Hamburg, S. 1 - 30, erschienen ist (ISSN 0938-068X, ISBN 3-928164-61-9), und M. L. Derrien in: L. Cerveny (Hrsg.), Stud. Surf. Sci. Catal., Bd. 27, S. 613 - 666, Elsevier, Amsterdam 1986.

Üblicherweise ist in C2-Strömen von Steamcrackern die Nebenkomponente Acetylen unerwünscht und in C3-Strömen sind die Nebenkomponenten Propin und Allen unerwünscht

Bei Kohlenwasserstoffströmen, die einem FCC-Cracker oder einem Reformer statt einem Steamcracker entstammen, treten analoge Probleme auf. Eine allgemeine Übersicht über solche Probleme gaben beispielsweise J. P. Boitiaux, C. J. Cameron, J. Cosyns, F. Eschard und P. Sarrazin in ihrem Vortrag "Selective Hydrogenation Catalysts and Processes: Bench to Industrial Scale" auf der DGMK-Konferenz "Selective Hydrogenation and Dehydrogenation" am 11. und 12. November 1993 in Kassel, Deutschland, dessen Manuskript auch im Tagungsbericht 9305 der DGMK Deutsche Wissenschaftliche Gesellschaft für Erdöl, Erdgas und Kohle e. V., Hamburg, S. 49 - 57, erschienen ist (ISSN 0938-068X, ISBN 3-928164-61-9).

Im Allgemeinen sind daher aus C2- und C3-Kohlenwasserstoffströmen zumeist ungesättigte Verbindungen mit Dreifachbindungen (Alkine, speziell Acetylen und Propin, letzteres auch als "Methylacetylen" bezeichnet) und/oder -bei C3-Strömen - ungesättigte Verbindungen mit mehr als einer Doppelbindung (Alkadiene, speziell Propadien, auch als "Allen" bezeichnet) zu entfernen, um die gewünschten Produkte wie Ethylen und/oder Propylen in der geforderten Qualität zu erhalten.

Die Entfernung unerwünschter ungesättigter Verbindungen aus diese enthaltenden Kohlenwasserstoffströmen geschieht häufig durch Selektivhydrierung mancher oder aller der unerwünschten ungesättigten Verbindungen im entsprechenden Kohlenwasserstoffstrom, vorzugsweise durch Selektivhydrierung zu nicht störenden, höher gesättigten Verbindungen und in besonders bevorzugter Weise zu den Wertprodukte darstellenden Komponenten des Kohlenwasserstoffstroms. Beispielsweise wird in C2-Strömen Acetylen zu Ethylen hydriert und in C3-Strömen Propin und Propadien zu Propylen.

Typischerweise sind solche Verbindungen vollständig oder mindestens bis auf Restgehalte von wenigen Gew.-ppm zu entfernen. Die ("Über-")Hydrierung zu Verbindungen, die höher gesättigt ist als das gewünschte Wertprodukt und/oder die parallele Hydrierung eines eine oder mehrere Mehrfachbindungen enthaltenden Wertprodukts zur entsprechenden höher oder vollständig gesättigten Verbindung sollen aufgrund des damit verbundenen Wertverlusts jedoch möglichst vermieden werden. Die Selektivität der Hydrierung der unerwünschten ungesättigten Verbindungen muß daher möglichst hoch sein. Zusätzlich sind im allgemeinen eine ausreichend hohe Aktivität des Katalysators und eine lange Standzeit erwünscht. Gleichzeitig soll der Katalysator möglichst auch keine anderen unerwünschten Nebenreaktionen bewirken. Üblicherweise werden Edelmetall-Trägerkatalysatoren eingesetzt, in denen Edelmetall auf einem Katalysatorträger abgeschieden ist. Häufig wird Palladium als Edelmetall verwendet, der Träger ist im allgemeinen ein poröses anorganisches Oxid, beispielsweise Kieselerde, Alumosilikat, Titandioxid, Zirkoniumdioxid Zinkaluminat, Zinktitanat, Spinelle und/oder Mischungen solcher Träger, meist werden jedoch Aluminiumoxid oder Siliciumdioxid verwendet. Weiterhin können Promotoren oder andere Zusatzstoffe enthalten sein. Verfahren zur selektiven Hydrierung ungesättigter Verbindungen in diese enthaltenden Kohlenwasserstoffströmen sind sowohl als Flüssigphasenhydrierung oder gemischte Gas/Flüssigphasenhydrierung, in Riesel- oder Sumpffahrweise, wie auch als reine Gasphasenhydrierung bekannt, wobei verschiedene verfahrenstechnische Maßnahmen zur Verbesserung der Selektivität publiziert wurden.

Beispielsweise lehrt EP-A 87 980 ein derartiges Verfahren in einem Festbettreaktor, bei dem der Hydrierwasserstoff an mindestens zwei Stellen entlang des Reaktors zugeführt wird, wodurch eine höhere Selektivität erreicht wird. EP-A 81 041 lehrt, daß der Zusatz von Kohlenmonoxid die Hydrier- und Isomerisierungaktivität des als Katalysatormetall verwendeten Palladiums herabsetzt und so die Selektivität erhöht. JP-A 01-110 594 lehrt den Zusatz weiterer Elektronendonor-Verbindungen, entweder in Form einer Dotierung des Katalysators, etwa mit Alkalimetallen, oder in Form eines Zusatzes zum Reaktionsgemisch, etwa von Alkoholen, Ethern oder stickstoffhaltigen Verbindungen.

Auch die Verwendung von Promotoren oder Dotierstoffen zusätzlich zum eigentlich hydrieraktiven Katalysatormetall ist bekannt.

So lehren J. P. Boitiaux, J. Cosyns, M. Derrien und G. Léger in Hydrocarbon Processing, 1985 (3), S.51-59, die Verwendung bimetallischer Katalysatoren, insbesondere solcher, die Metalle der Gruppe VIII (aktuelle IUPAC-Nomenklatur: Gruppen 8, 9 und 10), speziell Palladium, und Metalle der Gruppe IB (aktuelle IUPAC-Nomenklatur: Gruppe 11) des Periodensystems der Elemente enthalten. EP-A 564 328 und EP-A 564 329 lehren die Verwendung von Katalysatoren, die Metalle der Gruppe VIII, speziell Palladium, und Metalle der Gruppe IIIA (aktuelle IUPAC-Nomenklatur: Gruppe 3), speziell Indium oder Gallium, enthalten, und ihre Verwendung. EP-A 89 252 offenbart ein Verfahren zur Herstellung eines Palladium und Gold enthaltenden Trägerkatalysators und dessen Anwendung. DE-A 21 56 544 lehrt einen Palladium und Zink auf einem Kieselsäureträger enthaltenden Katalysator. EP-A 722 776 offenbart einen besonders gegen Verunreinigungen durch Schwefel resistenten Katalysator, der aus Palladium, mindestens einem Alkalifluorid und optional Silber auf einem anorganischen Träger wie TiO₂, ZrO₂ oder vorzugsweise Al₂O₃ besteht. EP-A 738 540 lehrt einen Katalysator, der Palladium, Silber, Alkali und Fluorid auf einem Aluminiumoxidträger enthält, wobei das Verhältnis von Fluorid zu Alkali zwischen 1,3 zu 1 bis 4 zu 1 beträgt.

Es ist auch möglich, die Eigenschaften des verwendeten Katalysators nicht nur durch verfahrenstechnische Maßnahmen oder die Verwendung bestimmter Zusatzstoffe zu beeinflussen, sondern auch durch die Art des Trägers und die Art der Verteilung der Aktivmasse auf der inneren und äußeren Oberfläche des Trägers.

So lehrt DE-A 20 59 978 Palladiumkatalysatoren auf einem Tonerdeträger ("Tonerde" ist ein gebräuchliches Synonym für Aluminiumoxid). Der Träger weist eine BET-Oberfläche von rund 120 m²/g auf und wird vor Abscheidung des Palladiums zunächst einer Wasserdampf-Behandlung bei 110-300 °C unterzogen und anschließend bei 500-1200 °C kalziniert.

DE-A 31 19 850 offenbart die Verwendung eines Katalysators, der aus Palladium und Silber auf einem Träger aus SiO₂ mit einer BET-Oberfläche im Bereich von 10 bis 200 m²/g oder auf einem Träger aus Al₂O₃ mit einer BET-Oberfläche von weniger als 100 m²/g besteht. Das Gewichtsverhältnis von Silber zu Palladium liegt dabei allgemein im Bereich von 0,1 bis 20 und in speziellen Ausführungsformen im Bereich von 0,7 bis 3 oder 1 bis 2,5. EP-A 780 155 und EP-A 686 615 offenbaren Katalysatoren, die aus Palladium und einem Metall der Gruppe IB des Periodensystems der Elemente auf einem Al₂O₃-Träger bestehen, wobei mindestens je 80 % des Palladiums und des Metalls der Gruppe IB in den Volumenteilen des Katalysatorkorns konzentriert sind, die vom Radius des Katalysatorkorns und einer Strecke vom Mittelpunkt, die 0,8 mal diesem Radius entspricht, begrenzt werden. Beim Katalysator von EP-A 780 155 liegen darüber hinaus das Metall der Gruppe IB und Palladium in einem Gewichtsverhältnis im Bereich von 0,4 bis 3 vor, und beim Katalysator von EP-A 686 615 in einem Gewichtsverhältnis im Bereich von 0,05 bis 0,4. EP-A 64 301 lehrt dagegen einen Palladium und Silber enthaltenden Katalysator, bei dem lediglich das Palladium in einer oberflächennahen Schicht angereichert ist und das Silber über das Katalysatorvolumen gleichverteilt ist, und bei dem Silber und Palladium in einem Gewichtsverhältnis von mindestens 2 vorliegen.

Die Anforderungen an Katalysatoren und Verfahren zur selektiven Hydrierung unerwünschter ungesättigter Verbindungen in diese enthaltenden Kohlenwasserstoffströmen im Hinblick auf die Verringerung des Restgehalts an unerwünschten ungesättigten Verbindungen nach der Hydrierung und auf die Erhöhung der Selektivität steigen ständig. Die bekannten Verfahren und Katalysatoren arbeiten zwar schon auf sehr hohem technischem Stand, sind aber angesichts der steigenden Anforderungen immer noch unbefriedigend. Es bestand daher die Aufgabe, einen verbesserten Katalysator und ein verbessertes Verfahren zur selektiven Hydrierung ungesättigter Verbindungen in diese enthaltenden Kohlenwasserstoffströmen und insbesondere zur Selektivhydrierung von Alkinen und/oder Alkadienen in diese enthaltenden C2- oder C3-Kohlenwasserstoffströmen zu finden.

Dementsprechend wurde ein Katalysator gefunden, der mindestens ein Metall der 10. Gruppe des Periodensystems der Elemente und mindestens ein Metall der 11. Gruppe des Periodensystems der Elemente auf einem Aluminiumoxidträger enthält, wobei das Metall oder die Metalle der 10. Gruppe im wesentlichen in einer oberflächennahen Randschicht des Katalysatorkorns konzentriert ist oder sind, das Metall oder die Metalle der 11. Gruppe im wesentlichen gleichmäßig über das Volumen des Katalysatorkorns verteilt vorliegt oder vorliegen, und das Gewichtsverhältnis des Metalls oder der Metalle der 11. Gruppe zum Metall oder zu den Metallen der 10. Gruppe höchstens 1,95 beträgt.

Weiterhin wurden ein Verfahren zur Herstellung dieses Katalysators sowie Verfahren zur selektiven Hydrierung ungesättigter Verbindungen in diese enthaltenden Kohlenwasserstoffströmen unter Verwendung des erfindungsgemäßen Katalysators gefunden.

Der erfindungsgemäße Katalysator weist besonders in der selektiven Hydrierung von Alkinen und Alkadienen zu Alkenen, hervorragende Eigenschaften auf, insbesondere eine hohe Selektivität sowohl bei Durchführung des Verfahrens in der gemischten Flüssig- und Gasphase als auch in der reinen Gasphase. Bei Verwendung des erfindungsgemäßen Katalysators kann die unerwünschte Überhydrierung zu den gesättigten Kohlenwasserstoffen wie Ethan oder Propan verringert werden. Zusätzlich ist der Katalysator vergleichsweise aktiv und kann über vergleichsweise lange Zeitdauern betrieben werden. Der erfindungsgemäße Katalysator zeigt diese vorteilhaften Eigenschaften auch ohne weitere verfahrenstechnische Maßnahmen, beispielsweise ohne Zusatz von Kohlenmonoxid oder Alkoholen, Ethern oder stickstoffhaltigen Verbindungen. Der erfindungsgemäße Katalysator ist besonders zur Verwendung als Katalysator der selektiven Hydrierung von Acetylen in C2-Strömen zu Ethylen oder als Katalysator der selektiven Hydrierung von Propin und Propadien in C3-Strömen zu Propylen geeignet.

Der Träger des erfindungsgemäßen Katalysators besteht im wesentlichen aus Aluminiunioxid, das neben unvermeidbaren Verunreinigungen in gewissem Umfang auch andere Zusätze enthalten kann. Beispielsweise können andere anorganische Oxide wie Oxide von Metallen der 2., 3., 4., 13. und 14. Gruppe des Periodensystems der Elemente enthalten sein, insbesondere Siliciumdioxid, Titandioxid, Zirkondioxid, Zinkoxid, Magnesiumoxid und Calciumoxid. Im Allgemeinen liegt der Gehalt an solchen, von Aluminiumoxid verschiedenen Oxiden unterhalb von 50 Gew.-%, beispielsweise unterhalb von 30 Gew.-% und bevorzugterweise unterhalb von 10 Gew.-%. In besonders bevorzugter Weise enthält der Träger neben Aluminiumoxid lediglich unvermeidbare Verunreinigungen. Als Aluminiumoxid werden die bekannten Aluminiumoxidphasen oder die bekannten teilweise hydratisierten Aluminiumoxidphasen verwendet, beispielsweise α-, β-, γ-, δ-, θ- oder κ-Aluminiumoxid, Böhmit, Pseudoböhmit oder ihre Gemische.

BET-Oberfläche, Porenvolumen und Porenradienverteilung (der Anteil des Porenvolumens als Funktion der Porendurchmesser) des Trägers des erfindungsgemäßen Katalysators werden im Hinblick auf den konkreten Anwendungsfall in dem Fachmann bekannter Weise optimiert. Die BET-Oberfläche des Trägers liegt im allgemeinen zwischen 2 und 200 m²/g. Sie beträgt beispielsweise mindestens 3 m²/g und bevorzugterweise mindestens 4 m²/g, sowie beispielsweise höchstens 100 m²/g, bevorzugterweise höchstens 70 m²/g und in besonders bevorzugter Form höchstens 10 m²/g. Das gesamte Porenvolumen des Trägers beträgt üblicherweise mindestens 0,1 ml/g, vorzugsweise mindestens 0,2 und besonders bevorzugt mindestens 0,25 ml/g sowie höchstens 1,0 ml/g, vorzugsweise höchstens 0,5 ml/g und besonders bevorzugt höchstens 0,4 ml/g. Im Allgemeinen liegen höchstens 10 Vol.-%, bevorzugterweise höchstens 8 Vol.-%, des gesamten Porenvolumens in Form von Poren mit einem mittleren Durchmesser von höchstens 60 Nanometer vor. Ferner beträgt der mittlere Porendurchmesser üblicherweise mindestens 50 Nanometer, bevorzugterweise mindestens 70 Nanometer und höchstens 150 Nanometer, bevorzugterweise höchstens 130 Nanometer.

Der Katalysatorträger wird in dem Fachmann bekannter Weise hergestellt, geeignete Träger sind auch kommerziell erhältlich. Zur Herstellung des Trägers wird üblicherweise ein geeigneter aluminiumhaltiger Rohstoff verformt, gegebenenfalls nach Peptisierung mit einem Peptisierungsmittel wie Wasser, verdünnter Säure oder verdünnter Base, danach werden die Formlinge getrocknet und kalziniert. Oberfläche und Porenstruktur des Trägers werden bekanntlich im wesentlichen durch die Trocknungs- und Kalzinationsbedingungen gemeinsam mit der Art des eingesetzten Rohstoffs und etwaigen zugesetzten Ausbrennstoffen wie Polymeren, Faserstoffen, natürlichen Ausbrennstoffen wie Nußschalenmehle oder anderen üblichen Zusätzen bestimmt. Die Form der Trägerpartikel ist nicht wesentlich, es können alle bekannten Formen wie beispielsweise Extrudate, Ringe, Zylinder, Hohlzylinder, Formkörper mit sternförmigem Querschnitt, Ringe mit Stegen ("Wagenräder") oder Kugeln verwendet werden.

Der Katalysator enthält mindestens ein Metall der 10. Gruppe des Periodensystems der Elemente und mindestens ein Metall der 11. Gruppe des Periodensystems der Elemente. Er kann darüber hinaus noch weitere Zusatzstoffe und/oder Promotoren enthalten.

Die Bezifferung der Gruppen des Periodensystems der Elemente richtet sich nach der aktuellen Numerierung der Internationalen Union für Reine und Angewandte Chemie (IUPAC). Die 10. Gruppe umfaßt die Elemente Nickel, Palladium und Platin und die 11. Gruppe die Elemente Kupfer, Silber und Gold. Der Katalysator enthält als Metall der 10. Gruppe Nickel, Palladium und/oder Platin, beispielsweise Palladium, in bevorzugter Weise als einziges Metall dieser Gruppe, und als Metall der 11. Gruppe Kupfer, Silber und/oder Gold, beispielsweise Silber, in bevorzugter Weise als einziges Metall dieser Gruppe. In bevorzugter Weise enthält der Katalysator Palladium und Silber, und in besonders bevorzugter Weise besteht die Aktivmasse des Katalysators aus Palladium und Silber.

Die Metalle können in reiner metallischer Form vorliegen, aber auch in Form von Verbindungen, beispielsweise in Form von Metalloxiden. Unter den Betriebsbedingungen eines Hydrierverfahrens liegen sie im allgemeinen in Form von Metallen vor. Die Umwandlung etwaiger Oxide in Metalle kann in bekannter Weise vor dem Einsatz des Katalysators in einem Hydrierverfahren durch Vorreduzierung und, falls für Manipulationen mit dem vorreduzierten Katalysator nötig, anschließende oberflächliche Passivierung erfolgen.

Das Metall oder die Metalle der 10. Gruppe ist oder sind im wesentlichen in einer oberflächennahen Randschicht des Trägers angereichert. Im Allgemeinen sind mehr als 80 Gew.-%, vorzugsweise mehr als 90 Gew.-% und in besonders bevorzugter Weise mehr als 95 Gew.-% des Metalls oder der Metalle in einer Schicht von nicht mehr als 0,6 Millimeter Dicke, die von der geometrischen Oberfläche des Katalysatorpartikels begrenzt wird, enthalten. Vorzugsweise ist diese Schicht nicht dicker als 0,45 Millimeter und in besonders bevorzugter Weise nicht dicker als 0,3 Millimeter.

Das Metall oder die Metalle der 11. Gruppe ist oder sind im wesentlichen gleichmäßig über das Volumen des Katalysatorkorns verteilt. Vorzugsweise ist der Gehalt des Katalysators an Metall oder Metallen der 11. Gruppe in jedem einzelnen repräsentativen Volumenelement eines Katalysatorkorns stets höher als das 0,3-fache und niedriger als das zweifache, und in besonders bevorzugter Weise höher als das 0,6-fache und niedriger als das 1,4-fache des integralen Gehalts des Katalysators an Metall oder Metallen der 11. Gruppe. Unter einem repräsentativem Volumenelement ist dabei ein Volumenelement zu verstehen, das zwar kleiner ist als das gesamte Katalysatorkorn, aber als Volumenelement noch die makroskopischen Eigenschaften des gesamten Korns, insbesondere dasselbe spezifische Porenvolumen, dieselbe Porenradienverteilung und dieselbe spezifische Oberfläche aufweist.

Das Gewichtsverhältnis des Metalls oder der Metalle der 11. Gruppe zum Metall oder zu den Metallen der 10. Gruppe beträgt höchstens 1,95, beispielsweise höchstens 1,9, bevorzugterweise höchstens 1,85 und in besonders bevorzugter Weise höchstens 1,8. Es beträgt weiterhin vorteilhafterweise mindestens 0,45. Es beträgt beispielsweise mindestens 0,5, bevorzugterweise mindestens 0,8 und in besonders bevorzugter Weise mindestens 1,6.

Der Gehalt des Katalysators an Metall oder Metallen der 10. Gruppe des Periodensystems der Elemente beträgt im allgemeinen mindestens 0,005 Gew.-%, bezogen auf seine Gesamtmasse, bevorzugterweise mindestens 0,01 Gew.-% und in besonders bevorzugter Weise mindestens 0,02 Gew.-%. Im Allgemeinen liegt dieser Gehalt bei höchstens 1 Gew.-%, bevorzugterweise bei höchstens 0,5 Gew.-% und in besonders bevorzugter Weise bei höchstens 0,1 Gew.-%. Niedrigere oder höhere Gehalte sind zwar möglich, aber im Normalfall wegen zu niedriger Aktivität oder zu hohen Rohstoffkosten wirtschaftlich unbefriedigend.

Beispielsweise kann der erfindungsgemäße Katalysator 0,025 oder 0,03 Gew.-% Palladium enthalten.

Der Gehalt des Katalysators an Metall oder Metallen der 11. Gruppe des Periodensystems der Elemente bemißt sich automatisch anhand des Gehalts an Metall oder Metallen der 10. Gruppe gemäß dem einzustellenden Gewichtsverhältnis zum Metall oder zu den Metallen der 10. Gruppe. Der Katalysator enthält daher vorteilhafterweise nicht mehr als 3,9 Gew.-%, bezogen auf seine Gesamtmasse, an Metall oder Metallen der 11. Gruppe, und vorteilhafterweise mindestens 0,00225 Gew.-%.

Der Katalysator kann, sofern erforderlich oder gewünscht, in seiner Aktivmasse zusätzlich auch andere Elemente als die Metalle der 10. und 11. Gruppen des Periodensystems der Elemente enthalten. Er kann insbesondere übliche Promotoren enthalten. Häufig verwendete Promotoren sind beispielsweise die Alkali- und Erdalkalimetalle wie Lithium, Natrium, Kalium, Rubidium, Cäsium, Magnesium, Calcium, Strontium und/oder Barium, aber auch die Elemente der 3. Gruppe wie Gallium und/oder Indium, oder andere Promotoren wie beispielsweise Zink oder Fluorid. Art und Menge solcher Promotoren sind im Einzelfall in üblicher Weise zu optimieren, im allgemeinen werden solche Promotoren im Bereich von einigen Gew.-ppm bis zu einigen Tausend Gew.-ppm zugesetzt.

Die auf dem Träger abzuscheidenden Metalle und gegebenenfalls Promotoren können mit jedem bekannten Verfahren auf den Träger aufgebracht werden, das zur Gleichverteilung des Metalls oder der Metalle der 11. Gruppe im Volumen des Katalysatorkorns bei gleichzeitiger Anreicherung des Metalls oder der Metalle der 10. Gruppe in einer oberflächennahen Randschicht des Katalysatorkorns führt. Die bevorzugte Methode ist jedoch die Tränkung mit einer Lösung der abzuscheidenden Substanzen und/oder Verbindungen, die sich im Zuge der weiteren Katalysatorherstellung in die abzuscheidenden Substanzen umwandeln. Tränkverfahren zur Abscheidung von Aktivkomponenten, Zusatzstoffen und/oder Dotierstoffen auf einem Träger, auch in ungleichmäßiger Verteilung über das Volumen des Katalysatorkorns, sind dem Fachmann bekannt. Die einzelnen abzuscheidenden Substanzen können einzeln und/oder in Teilmengen in mehreren Verfahrensschritten oder gemeinsam und vollständig in einem Verfahrensschritt abgeschieden werden. Bevorzugt ist die gemeinsame Abscheidung in einer Tränkstufe. Im Anschluß an die Tränkung oder nach den einzelnen Tränkstufen wird der getränkte Träger getrocknet, und durch Kalzinierung sowie gegebenenfalls andere bekannte Nachbehandlungsmethoden (beispielsweise Aktivierung und anschließende oberflächliche Passivierung) zum einsatzbereiten Katalysator umgewandelt.

Die gleichmäßige Verteilung des Metalls oder der Metalle der 11. Gruppe im Volumen des Katalysatorkorns bei gleichzeitiger Anreicherung des Metalls oder der Metalle der 10. Gruppe in einer oberflächennahen Randschicht des Katalysatorkorns erfordert die Einhaltung bestimmter Parameter während der Tränkung und der anschließenden Trocknung und gegebenenfalls Kalzinierung. Wichtige Parameter, die Gleichverteilung oder Anreicherung verschiedener Metalle entsprechend unterschiedlich beeinflussen, sind bekanntlich beispielsweise der pH-Wert der Tränklösung und die Trocknungstemperatur.

Im Allgemeinen wird der Träger mit einer Lösung von Salzen der abzuscheidenden Komponenten getränkt, wobei das Volumen der Lösung so bemessen wird, daß die Lösung praktisch vollständig vom Porenvolumen des Trägers aufgenommen wird ("incipient wetness"-Methode), wobei das Aufnahmevermögen des Trägers für die Tränklösung jedoch nicht voll ausgeschöpft werden muss, die Tränklösung kann also in einer Menge von weniger als 100 %, beispielsweise nicht mehr als 95 Vol.-%, nicht mehr als 90 Vol.-% oder nicht mehr als 85 Vol.-% des vom zu tränkenden Träger aufnehmbaren Flüssigkeitsvolumens verwendet werden. Die Konzentration der Salze in der Lösung wird so bemessen, daß nach Tränkung und Umwandlung des getränkten Trägers zum fertigen Katalysator die abzuscheidenden Komponenten in der gewünschten Konzentration auf dem Katalysator vorliegen. Die Salze werden so gewählt, daß sie keine bei der Katalysatorherstellung oder dessen späterer Verwendung störenden Rückstände hinterlassen. Meist werden Nitrate oder Ammoniumsalze verwendet.

Die Herstellung des erfindungsgemäßen Katalysators erfolgt vorzugsweise unter einstufiger Tränkung des Trägers nach der incipient wetness-Methode mit einer salpetersauren Lösung der Nitrate der abzuscheidenden Metalle. Die Konzentration der verwendeten Salpetersäure ist mindestens so hoch, daß eine klare Lösung vorliegt. Im Allgemeinen liegt der pH-Wert der Lösung bei höchstens 5 und bevorzugterweise bei höchstens 2.

Nach der Tränkung wird der getränkte Träger getrocknet, im allgemeinen bei einer Temperatur oberhalb von 50 °C und unterhalb von 120 °C. Vorzugsweise liegt die Trocknungstemperatur oberhalb von 60 °C und in besonders bevorzugter Weise oberhalb von 70 °C, sowie vorzugsweise unterhalb von 110 °C und in besonders bevorzugter Weise unterhalb von 100 °C. Sie liegt beispielsweise bei einer Temperatur von oder um 80 °C. Die Trocknung wird fortgesetzt, bis im getränkten Träger vorhandenes Wasser im wesentlichen vollständig entwichen ist, was im allgemeinen nach einigen Stunden der Fall ist. Übliche Trocknungsdauern liegen im Bereich von einer bis 30 Stunden und sind von der eingestellten Trocknungstemperatur abhängig, höhere Temperatur verkürzt die Trocknungszeit. Die Trocknung kann durch Anwenden eines Unterdrucks weiter beschleunigt werden.

Der getrocknete Katalysator ist im Prinzip einsatzbereit zu sei-ner Verwendung, die abgeschiedenen Metallverbindungen können direkt im Hydrierreaktor durch Behandlung mit Wasserstoff oder einem Wasserstoff enthaltenden Gas zu den Metallen reduziert werden. Vorzugsweise wird der Katalysator jedoch im Anschluß an die Trocknung noch kalziniert. Diese Kalzinierung dient im wesentlichen der Umwandlung der aufgetränkten Salze in die abzuscheidenden Komponenten oder Vorläufer solcher Komponenten. Im Falle der Auftränkung von Metallnitraten werden bei dieser Kalzinierung im wesentlichen die Nitrate in Metalle und/oder Metalloxide, die im Katalysator verbleiben, und nitrose Gase, die entweichen, zersetzt. Die nach der Kalzination noch auf dem Katalysator vorhandenen Metallverbindungen werden anschließend bei oder vor Verwendung des Katalysators zur Hydrierung im Reaktor automatisch bei der Hydrierung oder in bekannter Weise vorab zu den Metallen reduziert.

Die Kalzinationstemperatur beträgt im allgemeinen mindestens 200 °C und höchstens 650 °C. Sie beträgt vorzugsweise mindestens 300 °C und in besonders bevorzugter Weise mindestens 380 °C sowie vorzugsweise höchstens 550 °C und in besonders bevorzugter Weise höchstens 520 °C. Kalziniert wird im allgemeinen über mindestens 0,5 Stunden und höchstens 20 Stunden. In bevorzugter Weise wird über höchstens 10 Stunden und in besonders bevorzugter Weise über höchstens 5 Stunden Dauer kalziniert. Die Kalzination erfolgt in einem üblichen Ofen, beispielsweise in einem Drehrohrofen, in einem Bandkalzinierer oder in einem Kammerofen. Die Kalzination kann sich ohne zwischenzeitliche Abkühlung des getränkten und getrockneten Trägers direkt an die Trocknung anschließen.

Nach der Kalzination ist der Katalysator einsatzbereit. Falls er forderlich oder gewünscht, wird er vor seinem Einsatz zur Selektivhydrierung in bekannter Weise durch Vorreduzierung aktiviert und gegebenenfalls auch wieder oberflächlich passiviert.

Die erfindungsgemäßen Verfahren zur selektiven Hydrierung zeichnen sich durch den Einsatz des erfindungsgemäßen Katalysators aus. Die erfindungsgemäßen Hydrierverfahren unter Einsatz des erfindungsgemäßen Katalysators werden im allgemeinen genauso wie die bekannten, denselben Zwecken dienenden, heterogen katalysierten Hydrierverfahren durchgeführt. Sie können als heterogen katalysierte Gasphasenverfahren, bei denen sich sowohl der Kohlenwasserstoffstrom wie auch der Hydrierwasserstoff in der Gasphase befinden, oder als heterogen katalysierte Gas/Flüssigphasenverfahren, bei denen der Kohlenwasserstoffstrom zumindest teilweise in flüssiger Phase und Wasserstoff in der Gasphase und/oder in gelöster Form in der Flüssigphase vorliegen, durchgeführt werden. Die einzustellenden Parameter wie Durchsatz an Kohlenwasserstoffstrom, ausgedrückt als Raumgeschwindigkeit in der Einheit [m³/m³*h], bezogen auf das Katalysatorvolumen, Temperatur und Druck werden analog zu denen bekannter Verfahren gewählt.

Die Menge des eingesetzten Wasserstoffs, bezogen auf die Menge des zugeführten Kohlenwasserstoffstroms, ist abhängig von dem Gehalt des Kohlenwasserstoffstroms an unerwünschten ungesättigten Verbindungen und deren Art. Im Allgemeinen wird der Wasserstoff in einer Menge im Bereich vom 0,8 bis zum 5-fachen der stöchiometrisch zum vollständigen Wasserstoffumsatz beim Reaktordurchgang erforderlichen Menge zugegeben, vorzugsweise im Bereich vom 0,95 bis 2-fachen dieser Menge. Die Hydrierung von Dreifachbindungen läuft im Normalfall schneller ab als die konjugierter Doppelbindungen, und diese wiederum schneller als die unkonjugierter Doppelbindungen. Dies erlaubt eine entsprechende Steuerung des Verfahrens anhand der zugegebenen Wasserstoffmenge. Der Wasserstoff kann Inerte enthalten, beispielsweise Edelgase wie Helium, Neon oder Argon, andere inerte Gase wie Stickstoff, Kohlendioxid und/oder niedere Alkane, etwa Methan, Ethan, Propan und/oder Butan. Solche Inertgase im Wasserstoff liegen vorzugsweise in einer Konzentration von weniger als 30 Vol.-% vor. Bevorzugterweise ist der Wasserstoff frei von Kohlenmonoxid.

Die Verfahren können in einem oder in mehreren parallelen oder hintereinandergeschalteten Reaktoren, jeweils im einfachen Durchgang oder in Kreislauffahrweise durchgeführt werden. Bei Durchführung der Verfahren in der Gas-/Flüssigphase wird der Kohlenwasserstoffstrom nach Durchtritt durch einen Reaktor üblicherweise in einem Abscheider von Gasen befreit und ein Teil der erhaltenen Flüssigkeit in den Reaktor zurückgeführt. Das Verhältnis zwischen zurückgeführtem und erstmals in den Reaktor eingespeistem Kohlenwasserstoffstrom, das sogenannte Rücklaufverhältnis, wird so eingestellt, daß unter den sonstigen Reaktionsbedingungen, wie Druck, Temperatur, Durchsatz und Wasserstoffmenge, der gewünschte Umsatz erreicht wird.

Einsatzzwecke der erfindungsgemäßen Verfahren sind vor allem die Hydrierung von Alkinen und/oder Alkadienen zu Alkenen, insbesondere die selektive Hydrierung von Acetylen in C2-Strömen zu Ethylen bei minimaler Bildung von Ethan und die selektive Hydrierung von Propin und/oder Propadien in C3-Strömen zu Propylen bei minimaler Bildung von Propan.

Die selektive Hydrierung von Acetylen in C2-Strömen zu Ethylen wird üblicherweise als Gasphasenverfahren mit einer Raumgeschwindigkeit des gasförmigen C2-Stroms von 500 m³/m³*h, bezogen auf das Katalysatorvolumen, bis 10 000 m³/m³*h bei einer Temperatur von 0 °C bis 250 °C und einem Druck von 0,01 bar bis 50 bar durchgeführt, wobei je Mol Acetylen im C2-Strom mindestens ein Mol, bevorzugterweise mindestens 1,2 Mole, und höchstens zwei Mole, bevorzugterweise höchstens 1,8 Mole, Wasserstoff zugegeben werden.

Die selektive Hydrierung von Propin und/oder Propadien in C3-Strömen zu Propylen wird üblicherweise als Gasphasenverfahren oder als Gas-/Flüssigphasenverfahren mit einer Raumgeschwindigkeit des flüssigen C3-Stroms von 1 m³/m³*h, bezogen auf das Katalysatorvolumen, bis 50 m³/m³*h bei einer Temperatur von 0 °C bis 180 °C und einem Druck von 0,01 bar bis 50 bar durchgeführt, wobei je Mol Propin und Propadien im C3-Strom ein bis zwei Mole Wasserstoff zugegeben werden.

### Beispiele

### Beispiel 1: Herstellung von Katalysator 1

In einem Mischer wurde Boehmit (Versal® 250, bezogen von Euro Support, Amsterdam) mit Wasser angefeuchtet, in einem Kollergang intensiv bearbeitet, bis die Masse gut verformbar war, und anschließend zu 3 mm-Strängen extrudiert. Danach wurden die Stränge 2 Stunden bei 120 °C getrocknet und 2 Stunden bei 1100 °C kalziniert. Danach wurden die Stränge (BET-Oberfläche 100 m²/g) mit einer salpetersauren wäßrigen Lösung (pH-Wert 1,9) von Silbernitrat und Palladiumnitrat bei Raumtemperatur imprägniert. Die Menge der Tränklösung betrug 90 Vol.-% der vom verwendeten Träger maximal aufnehmbaren Menge, die Konzentrationen der Metallnitrate in der Tränklösung wurden so eingestellt, daß der Katalysator letztendlich 0,025 Gew.-% metallisches Palladium und 0,045 Gew.-% metallisches Silber enthielt. Das Gewichtsverhältnis von Silber zu Palladium betrug folglich 1,8. Der Kontakt wurde bei 80 °C getrocknet und anschließend bei 400 °C kalziniert. In Proben des fertigen Katalysators wurde durch Rasterelektronenmikroskopie (SEM-Analyse) und durch EPMA-Analyse (Electron Probe Micro Analysis) festgestellt, daß Silber im wesentlichen gleichverteilt und Palladium in einer oberflächennahen Randschicht angereichert vorlag.

Der so hergestellte Katalysator wurde als Katalysator 1 bezeichnet.

### Beispiel 2: Herstellung von Vergleichskatalysator V1

Beispiel 1 wurde wiederholt, wobei jedoch die Konzentrationen der Metallnitrate in der Tränklösung so eingestellt wurden, daß ein Metallgehalt des fertigen Katalysators von 0,03 Gew.-% Palladium und 0,2 Gew.-% Silber resultierte. Das Gewichtsverhältnis von Silber zu Palladium betrug folglich 6,7. In Proben des fertigen Katalysators wurde durch Rasterelektronenmikroskopie (SEM-Analyse) und durch EPMA-Analyse (Electron Probe Micro Analysis) festgestellt, daß Silber im wesentlichen gleichverteilt und Palladium in einer oberflächennahen Randschicht angereichert vorlag.

Der so hergestellte Katalysator wurde als Vergleichskatalysator V1 bezeichnet.

### Beispiel 3: Herstellung von Katalysator 2

In einem Mischer wurden zwei Aluminiumoxidpulver mit BET-Oberflächen von 217 und 251 m²/g, Rüttelgewichten von 803 und 1018 g/l und Glühverlusten von 3,7 und 25,1 Gew. -%, respektive, im Gewichtsverhältnis von 3 : 2 trocken miteinander vermischt,mit verdünnter Salpetersäure angefeuchtet und im Kollergang bis zur guten Verformbarkeit der Masse bearbeitet. Es wurden Stränge extrudiert, die bei 120 °C getrocknet und bei 1150 °C kalziniert wurden.

Danach wurden die Stränge (BET-Oberfläche 6 m²/g)mit einer salpetersauren wäßrigen Lösung (pH-Wert 1,9) von Silbernitrat und Palladiumnitrat bei Raumtemperatur imprägniert. Die Menge der Tränklösung betrug 90 Vol. -% der vom verwendeten Träger maximal aufnehmbaren Menge, die Konzentrationen der Metallnitrate in der Tränklösung wurden so eingestellt, daß der Katalysator letztendlich 0,025 Gew.-% metallisches Palladium und 0,045 Gew. -% metallisches Silber enthielt. Das Gewichtsverhältnis von Silber zu Palladium betrug folglich 1,8. Der Kontakt wurde bei 80 °C getrocknet und anschließend bei 400 °C kalziniert. In Proben des fertigen Katalysators wurde durch Rasterelektronenmikroskopie (SEM-Analyse) und durch EPMA-Analyse (Electron Probe Micro Analysis) festgestellt, daß Silber im wesentlichen gleichverteilt und Palladium in einer oberflächennahen Randschicht angereichert vorlag.

Der so hergestellte Katalysator wurde als Katalysator 2 bezeichnet.

### Beispiel 4: Herstellung von Katalysator 3

Beispiel 3 wurde wiederholt, wobei jedoch die Konzentrationen der Metallnitrate in der Tränklösung so eingestellt wurden, daß ein Metallgehalt des fertigen Katalysators von 0,03 Gew.-% Palladium und 0,045 Gew.-% Silber resultierte. Das Gewichtsverhältnis von Silber zu Palladium betrug folglich 1,5. In Proben des fertigen Katalysators wurde durch Rasterelektronenmikroskopie (SEM-Analyse) und durch EPMA-Analyse (Electron Probe Micro Analysis) festgestellt, daß Silber im wesentlichen gleichverteilt und Palladium in einer oberflächennahen Randschicht angereichert vorlag.

Der so hergestellte Katalysator wurde als Katalysator 3 bezeichnet.

### Beispiel 5: Herstellung von Vergleichskatalysator V2

Beispiel 3 wurde wiederholt, wobei jedoch die Konzentrationen der Metallnitrate in der Tränklösung so eingestellt wurden, daß ein Metallgehalt des fertigen Katalysators von 0,05 Gew.-% Palladium und 0,025 Gew.-% Silber resultierte. Das Gewichtsverhältnis von Silber zu Palladium betrug folglich 2,0. In Proben des fertigen Katalysators wurde durch Rasterelektronenmikroskopie (SEM-Analyse) und durch EPMA-Analyse (Electron Probe Micro Analysis) festgestellt, daß Silber im wesentlichen gleichverteilt und Palladium in einer oberflächennahen Randschicht angereichert vorlag.

Der so hergestellte Katalysator wurde als Vergleichskatalysator V2 bezeichnet.

### Beispiel 6: Hydrierversuche

Die Katalysatoren 1, 2, 3 und die Vergleichskatalysatoren V1 und V2 wurden in einer Labor-Versuchsanlage mit einem Festbettreaktor bei Normaldruck auf ihre Eigenschaften bei der Hydrierung von Acetylen in einem Ethylenstrom geprüft. Dazu wurden jeweils an 66 ml des getesteten Katalysators ein Modell-Gasgemisch aus 99 Vol.-% Ethylen und 1 Vol.-% Acetylen hydriert. Diesem Gasgemisch wurde vor dem Reaktor Wasserstoff zugesetzt, so daß im dem Reaktor zugeführten Gasstrom ein molares Verhältnis von Wasserstoff zu Acetylen von 1,8 vorlag. Die Temperatur T des Reaktors wurde so eingestellt, daß ein Acetylenumsatz von 90 Mol.-% erreicht wurde. Die Analysen wurden mittels eines Gaschromatographen durchgeführt. Der jeweils eingestellte Durchsatz durch den Reaktor, ausgedrückt als Raumgeschwindigkeit des gesamten dem Reaktor zugeführten Gasstroms, bezogen auf das eingesetzte Katalysatorvolumen (gas hourly space velocity, GHSV), sowie die einzustellende Temperatur und die erreichten Selektivitäten S der Hydrierung von Acetylen zu Ethylen sind in der folgenden Tabelle wiedergegeben.

| Kat. | BET-Oberfläche [m2/g] | Gew. -Verhältnis Ag/Pd | GHSV [1/h] | T [°C] | S [Mol-%] |
|---|---|---|---|---|---|
| 1 | 100 | 1,8 | 5400 | 85 | 33 |
| V1 | 100 | 6,7 | 5400 | 107 | 40 |
| 2 | 6 | 1,8 | 3000 | 63 | 35 |
| 3 | 6 | 1,5 | 3000 | 65 | 21 |
| V2 | 6 | 2,0 | 3000 | 65 | 15 |

Der Vergleich von Katalysator 1 mit dem Vergleichskatalysator V1 zeigt, daß eine Erhöhung des Gewichtsverhältnisses von Silber zu Palladium zwar zu einer Selektivitätszunahme führen kann, aber gleichzeitig den Katalysator drastisch und in technisch äußerst nachteiliger und unwirtschaftlicher Weise inaktiver macht. Der Vergleich der Katalysatoren 2 und 3 mit dem Vergleichskatalysator V2 zeigt, daß mit dem erfindungsgemäßen Katalysator befriedigende Selektivitäten bei technisch vorteilhaften und wirtschaftlich befriedigenden Aktivitäten erreichbar sind.

## Patentansprüche

1. Katalysator, der mindestens ein Metall der 10. Gruppe des Periodensystems der Elemente und mindestens ein Metall der 11. Gruppe des Periodensystems der Elemente auf einem Aluminiumoxidträger enthält, wobei das Metall oder die Metalle der 10. Gruppe im wesentlichen in einer oberflächennahen Randschicht des Katalysatorkorns konzentriert ist oder sind, das Metall oder die Metalle der 11. Gruppe im wesentlichen gleichmäßig über das Volumen des Katalysatorkorns verteilt vorliegt oder vorliegen, und das Gewichtsverhältnis des Metalls oder der Metalle der 11. Gruppe zum Metall oder zu den Metallen der 10. Gruppe höchstens 1,95 beträgt.

2. Katalysator nach Anspruch 1, der das Metall oder die Metalle der 10. Gruppe in einer Menge von mindestens 0,005 Gew.-% und höchstens 2 Gew.-%, bezogen auf das Gesamtgewicht des Katalysators, enthält.

3. Katalysator nach Anspruch 1, der als Metall der 10. Gruppe Palladium enthält.

4. Katalysator nach Anspruch 1, der als Metall der 11. Gruppe Silber enthält.

5. Katalysator nach Anspruch 1, der als Metall der 10. Gruppe Palladium und als Metall der 11. Gruppe Silber enthält.

6. Verfahren zur Herstellung des in den Ansprüchen 1 bis 5 beschriebenen Katalysators durch Tränkung eines Aluminiumoxidträgers mit einer wäßrigen salpetersauren Lösung von Salzen der Metalle der Gruppen 10 und 11, Trocknung bei einer Temperatur im Bereich von 60 °C bis 110 °C und Kalzination bei einer Temperatur im Bereich von 350 °C bis 550 °C.

7. Verwendung des in den Ansprüchen 1 bis 5 beschriebenen Katalysators zur Hydrierung ungesättigter Verbindungen.

8. Verwendung des in den Ansprüchen 1 bis 5 beschriebenen Katalysators zur selektiven Hydrierung von Alkinen und Alkadienen zu Alkenen.

9. Verfahren zur selektiven Hydrierung ungesättigter Verbindungen in Kohlenwasserstoffströmen in der Gas- oder Gas/Flüssigphase bei Temperaturen im Bereich von 0 °C bis 250 °C und Drücken im Bereich von 0,01 bis 50 bar, dadurch gekennzeichnet, daß man die selektive Hydrierung in Gegenwart des in den Ansprüchen 1 bis 5 beschriebenen Katalysators durchführt.

10. Verfahren nach Anspruch 9, dadurch gekennzeichnet, daß man Acetylen in einem C2-Strom selektiv zu Ethylen hydriert.

11. Verfahren nach Anspruch 9, dadurch gekennzeichnet, daß man Propin und/oder Propadien in einem C3-Strom selektiv zu Propylen hydriert.
